(19) Europäisches Patentamt
European Patent Office
Office européen des brevets

(11) **EP 4 660 609 A1**

(12) **EUROPEAN PATENT APPLICATION**
published in accordance with Art. 153(4) EPC

(43) Date of publication:
**10.12.2025 Bulletin 2025/50**

(21) Application number: **24750158.8**

(22) Date of filing: **26.01.2024**

(51) International Patent Classification (IPC):
*G01N 21/27* (2006.01)   *A23D 9/00* (2006.01)
*A23D 9/02* (2006.01)   *A47J 37/12* (2006.01)
*B01D 24/00* (2006.01)   *B01D 29/00* (2006.01)
*B01D 35/02* (2006.01)   *F01M 11/10* (2006.01)
*F16N 39/06* (2006.01)   *G01J 3/46* (2006.01)
*G01N 33/26* (2006.01)

(52) Cooperative Patent Classification (CPC):
A23D 9/00; A23D 9/02; A47J 37/12; B01D 24/00;
B01D 29/00; B01D 35/02; F01M 11/10;
F16N 39/06; G01J 3/46; G01N 21/27; G01N 33/26

(86) International application number:
**PCT/JP2024/002425**

(87) International publication number:
**WO 2024/162209 (08.08.2024 Gazette 2024/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **01.02.2023 JP 2023014037**

(71) Applicant: **NSK LTD.**
**Tokyo 141-8560 (JP)**

(72) Inventors:
• **INABA Takenobu**
**Fujisawa-shi, Kanagawa 251-8501 (JP)**
• **WATANABE Yuya**
**Fujisawa-shi, Kanagawa 251-8501 (JP)**

(74) Representative: **Grünecker Patent- und
Rechtsanwälte
PartG mbB
Leopoldstraße 4
80802 München (DE)**

(54) **METHOD FOR EVALUATING FILTRATION FILTER**

(57)   Provided is a method for evaluating a filtration filter that is simple, requires only a small amount of sampling, and allows immediate on-site evaluation of filtration performance and degradation inhibition performance, without the need for a large-scale detection device. This method for evaluating a filtration filter used for regenerating used fats and oils comprises: an imaging step for imaging fats and oils filtered through the filtration filter or diluted with a solvent, together with a color sample (1), by means of an imaging device; an image information acquisition step for acquiring, on the basis of image information for the imaged color sample (1), image information for the imaged fats and oils or fats and oils diluted with a solvent; a fat and oil degradation determination step for determining the degradation state of the fats and oils on the basis of the image information; and a filtration filter evaluation step for evaluating the filtration performance and the anti-degradation performance of the filtration filter on the basis of the determination result of the fat and oil degradation determination step.

*FIG. 1*

# EP 4 660 609 A1

**Description**

TECHNICAL FIELD

The present invention relates to a method for evaluating a filtration filter used for recycling fats and oils such as used lubricating oil and used edible oil.

BACKGROUND ART

Edible oil is used for various foods, but edible oil used for cooking deep-fried foods such as tempura and fries oxidatively deteriorates when the edible oil is heated during cooking or left unattended, causing a taste, an odor, and an appearance of foods to deteriorate. The viscosity of oxidatively deteriorated oil increases, leading to poor drainage. Therefore, the edible oil that has oxidatively deteriorated to a predetermined level or higher is discarded. Hence, from the viewpoint of global environment conservation, it is desired to delay the oxidative deterioration in the edible oil as much as possible to reduce the number of times the edible oil is discarded. In addition, by reducing the number of times the edible oil is discarded, the number of times of cleaning a deep-frying utensil (fryer) used for deep-fried foods is reduced, and this adds the benefit that the amount of water used for cleaning can be reduced.

Various edible oil filtration filters are used to recycle used edible oil, and for example, Patent Literature 1 describes an edible oil filtration filter in which an adsorbent is uniformly dispersed by mixing fibers such as pulp and an adsorbent such as activated carbon powder with water by stirring, and dehydration performed by compression molding.

The filtration capability of the edible oil filtration filter decreases with the number of times of filtration, and therefore, it is necessary to determine the replacement time, and the number of times of use is generally specified in consideration of safety. However, deterioration states differ for each used edible oil, and the filtration capabilities of the edible oil filtration filter also differ accordingly. Therefore, the designated number of times of use is not necessarily suitable.

In addition, in various mechanical devices, a filtration filter is used to recycle lubricating oil used in a lubricating location such as a rotation location, but there is a similar problem.

As a method for evaluating the filtration capability of a filtration filter, for example, in Patent Literature 2, a dispersion of metal particles is filtered through a filter, and a filtrate is sampled. The metal particles are dissolved in a chemical solution to form a solution, and the solution is analyzed by ICP-MS. The amount of the metal particles in the sampled filtrate is detected, and a filtration membrane is evaluated based on the detected amount.

CITATION LIST

PATENT LITERATURE

Patent Literature 1: JP4593127B
Patent Literature 2: JP6006541B

SUMMARY OF INVENTION

TECHNICAL PROBLEM

However, in the evaluation method described in Patent Literature 2, a dispersion of metal particles is required, and only the collection performance of foreign substances in the filtrate can be evaluated. It is necessary to use ICP-MS during the evaluation, the device is large-scale. In addition, it is necessary to subject the filtrate to the ICP-MS, and the filtration performance cannot be immediately evaluated.

Therefore, an object of the present invention is to provide a method for evaluating a filtration filter, which is capable of easily evaluating filtration performance and deterioration inhibiting performance immediately on site in a small sampling amount without using a large-scale detection device.

SOLUTION TO PROBLEM

The above object of the present invention is achieved by the following configuration [1] related to a method for evaluating a filtration filter.

[1] A method for evaluating a filtration filter, which is a method for evaluating a filtration filter used for recycling used fats and oils, the method including:

an imaging step of capturing an image of fats and oils filtered by the filtration filter or an image of the fats and oils diluted with a solvent, together with a color chart, by an imaging device;
an image information acquisition step of acquiring image information of the imaged fats and oils or image information of the fats and oils diluted with the solvent based on image information of the imaged color chart;
a fats and oils deterioration determination step of determining a deterioration state of the fats and oils based on the image information; and
a filtration filter evaluation step of evaluating filtration performance and deterioration inhibiting performance of the filtration filter based on a determination result of the fats and oils deterioration determination step.

A preferred embodiment according to the present invention related to the method for evaluating a filtration filter relates to the following [2] to [6].
[2] The method for evaluating a filtration filter according to claim [1],

in which the image information in the image information acquisition step is an RGB value of the fats and oils after filtration or an RGB value of the fats and oils diluted with the solvent, and
in the fats and oils deterioration determination step, lightness ($\Delta$E) defined by the following formula (1) and a maximum color difference are calculated from the RGB value to determine a correlation between the lightness ($\Delta$E) and the maximum color difference which are defined as follows:

• 

$$\bullet \text{Lightness } (\Delta E) = (R^2+G^2+B^2)^{0.5}\ldots(1); \text{ and}$$

• Maximum color difference: difference between maximum value and minimum value in RGB values.

...(1); and

[3] The method for evaluating a filtration filter according to [2], further including:

an image information correction step of correcting a white balance in the image information of the imaged fats and oils based on a white balance in the image information of the color chart, in a subsequent stage of the image information acquisition step,
in which in the fats and oils deterioration determination step, the deterioration state of the fats and oils is determined based on the corrected image information.

[4] The method for evaluating a filtration filter according to [3], in which the imaging device has a white balance correction function.
[5] The method for evaluating a filtration filter according to [4], in which the imaging device is a digital camera or a camera-equipped mobile terminal.
[6] The method for evaluating a filtration filter according to any one of [1] to [5], in which the fats and oils is lubricating oil or edible oil.

ADVANTAGEOUS EFFECTS OF INVENTION

According to the method for evaluating a filtration filter of the present invention, it is possible to easily evaluate filtration performance and deterioration inhibiting performance immediately on site in a small sampling amount without using a large-scale detection device.

BRIEF DESCRIPTION OF DRAWINGS

[FIG. 1] FIG. 1 is a top view illustrating an example of a color chart.
[FIG. 2] FIG. 2 is a schematic view illustrating a device used in Test 1 and Test 2.
[FIG. 3] FIG. 3 is a graph illustrating results of a commercially available filter in Test 1.

[FIG. 4] FIG. 4 is a graph illustrating results obtained by a deterioration inhibiting filter in Test 1.
[FIG. 5] FIG. 5 is a graph illustrating results obtained by a commercially available filter in Test 2.
[FIG. 6] FIG. 6 is a graph illustrating results obtained by a deterioration inhibiting filter in Test 2.

DESCRIPTION OF EMBODIMENTS

Hereinafter, an embodiment of the present invention will be described in detail with reference to the drawings. It should be noted that the present invention is not limited to the embodiments described below, and can be freely modified and implemented without departing from the gist of the present invention.

(Imaging Step)

In a method for evaluating a filtration filter of the present invention (hereinafter referred to as "evaluation method"), first, a device or equipment using fats and oils such as lubricating oil and edible oil, for example, a rolling bearing, a ball screw device, or a fryer is stopped, the fats and oils is sampled and taken into a container, and is imaged together with a color chart. Various imaging devices can be used for image capturing, and the type thereof is not particularly limited. For example, a digital camera or a camera-equipped mobile terminal such as a smartphone and a tablet can be used. The type of a light source during image capturing is not particularly limited, and detection can be performed at various places.

(Image Information Acquisition Step)

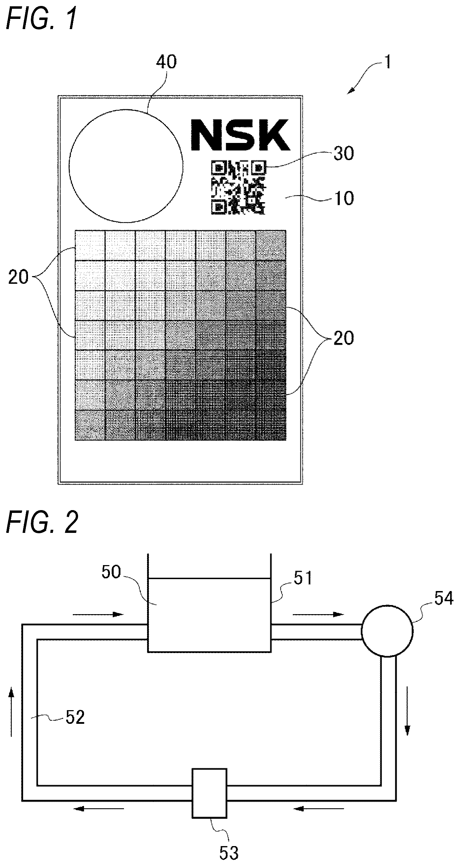

In this step, a color chart is used. As illustrated in FIG. 1, a color chart 1 is a list in which a plurality of color samples 20 having different hues and shades from white (upper left in the drawing) to black (lower right in the drawing) are arranged on a surface of a base sheet 10. The image information of the color chart 1 is printed on the base sheet 10 as an identification code 30. As the identification code 30, a bar code, a QR code (registered trademark) illustrated in the drawing, or the like is used.

Then, the sampled fats and oils is charged into a container (not illustrated) such as a petri dish or a transparent bin, placed on a sample placement portion 40 indicated by a circle in the drawing, and imaged together with the color chart 1 using various imaging devices. Here, a sampling amount of the fats and oils is about 10 mg, which is a trace quantity. The fats and oils may be used alone or may be diluted with a solvent. The solvent is not particularly limited as long as it is easily mixed with the fats and oils and has a colorless property and transparency to such an extent that there is no problem in capturing an image of the color of the fats and oils. Specifically, organic solvents, kerosene, and gasoline are preferable. The fats and oils discolored in a dark color, particularly in a black color can be more finely separated by diluting the fats and oils with a solvent.

(Image Information Correction Step)

The image information of the imaged fats and oils is compared with the image information of the color chart 1. At this time, it is preferable to correct the white balance in the image information of the imaged fats and oils. When the white balance is corrected, it is possible to make the image information of the imaged fats and oils more appropriately correspond to the image information of the color chart 1 regardless of the environment of the imaging location, that is, the detection location, which may be affected by brightness or the like.

When the imaging device to be used has a white balance correction function, the white balance correction can be performed by the white balance correction function. Alternatively, the image information obtained by the imaging device may be transmitted to an external processing device such as a server, and correction may be performed by a white balance correction function of the processing device. The comparison between the image information of the lubricant and the image information of the color chart 1, which will be described below, may be performed in the imaging device or may be performed in an external processing device.

(Fats and Oils Deterioration Determination Step)

Next, a hue of an image of the fats and oils with corrected white balance (hereinafter referred to as "corrected image") is determined. The hue is expressed by three colors, red (R), green (G), and blue (B), and lightness ($\Delta E$) is determined from the following formula (1) based on the RGB values of the corrected image.

$$\text{Lightness } (\Delta E) = (R^2 + G^2 + B^2)^{0.5} \ldots (1)$$

A difference between the maximum value and the minimum value of the RGB values is the maximum color difference of the corrected image of the lubricant.

As shown in test examples to be described below, when the lightness ($\Delta$E) and the maximum color difference are determined for each predetermined filtration time or for each number of times of filtration, and the lightness ($\Delta$E) and the maximum color difference are plotted in an X axis and a Y axis, respectively, and graphed, a semicircular arc-shaped correlation is observed. Based on this graph, the degree of deterioration (deterioration state) of the collected fats and oils is determined.

In the case where the fats and oils is diluted, the correction based on a dilution ratio can be performed using a correction table or the like created in advance through the correction function implemented in the imaging device or the server.

(Filtration Filter Evaluation Step)

Then, the filtration performance and the deterioration inhibiting effect of the filtration filter are evaluated based on the deterioration state of the fats and oils.

EXAMPLES

Hereinafter, the present invention will be further clarified with reference to Examples and Comparative Examples.

<Test 1: Evaluation of Continuous Filtration>

A commercially available filter was prepared as a comparative example, and a deterioration inhibiting filter was prepared as an example. The deterioration inhibiting filter is provided with an effect of inhibiting deterioration in oil by causing an antioxidant to adhere thereto.

Each filter was attached to the device shown in FIG. 2, and the deterioration state of rapeseed oil was examined. The illustrated device includes an oil tank 51 in which rapeseed oil 50 is stored, an annular pipe 52 through which the rapeseed oil 50 passes, a pump 54 that feeds the rapeseed oil 50, and a processing unit 53 in which a filtration filter is loaded. The rapeseed oil 50 in the oil tank 51 is fed through the pipe 52 by the pump 54, filtered in the processing unit 53, and then returned to the oil tank 51.

Then, the rapeseed oil 50 was continuously filtered while being heated to 180°C, and the filtered rapeseed oil 50 was collected after 24 hours, 48 hours, and 96 hours. The sampling amount was 1 g each time.

The sampled rapeseed oil was charged in a petri dish and placed on the sample placement portion 40 of the color chart 1 shown in FIG. 1, the sampled rapeseed oil and the color chart were imaged together by a digital camera with a white balance correction function, and RGB values were determined from the obtained corrected image. Then, the lightness ($\Delta$E) and the maximum color difference were calculated from the obtained RGB values based on the formula (1), and a graph in which the lateral axis represents the lightness ($\Delta$E) and the vertical axis represents the maximum color difference was created.

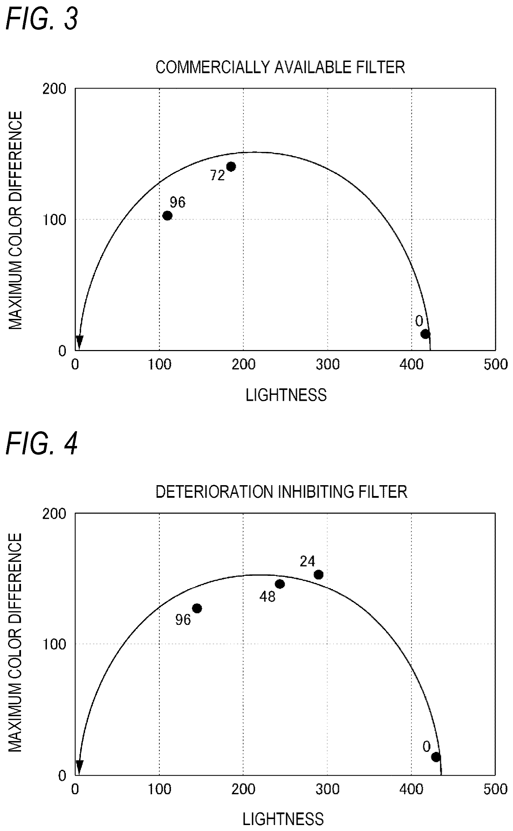

FIG. 3 is a graph showing the lightness ($\Delta$E) and the maximum color difference for each filtration time in the case of filtration with a commercially available filter, and FIG. 4 is a graph showing the lightness ($\Delta$E) and the maximum color difference for each filtration time in the case of filtration with a deterioration inhibiting filter. As shown in the drawings, in both filters, the rapeseed oil has almost no color and high lightness when it is unused oil. However, as the filtration time elapsed, a continuous change was observed in which the lightness decreased because the filtered rapeseed oil deteriorated and became brown, and the maximum color difference increased because the hue decreased. In addition, when the commercially available filter and the deterioration inhibiting filter are compared after 96 hours of filtration, it can be seen that the rapeseed oil filtered by the commercially available filter has both a lower lightness ($\Delta$E) and a lower maximum color difference than the rapeseed oil filtered by the deterioration inhibiting filter, and the deterioration has progressed.

<Test 2: Evaluation of Number of Times of Filtration>

The deteriorating rapeseed oil was filtered separately using a commercially available filter and a deterioration inhibiting filter. At this time, only oil was dropped from each filter, and the device shown in FIG. 2 was not used. Then, filtration was performed once or three times, and a change in hue of the rapeseed oil after filtration was observed. The sampling amount was 1 g each time, which is the same as that of Test 1.

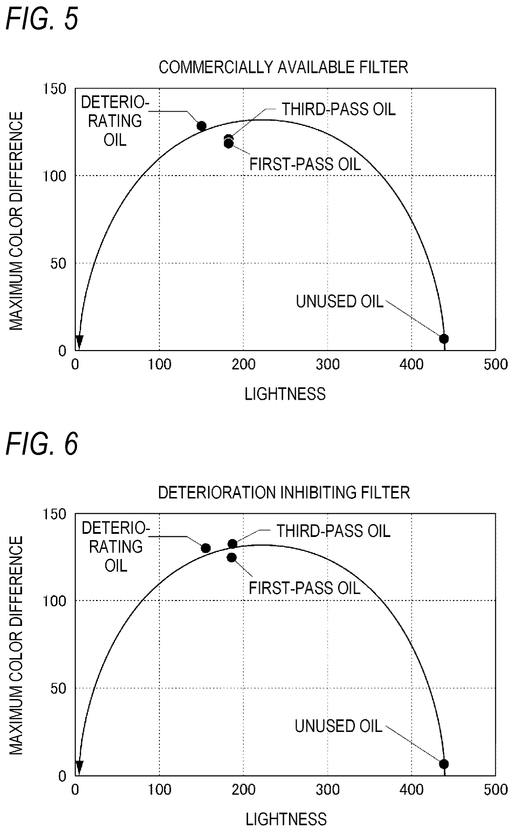

The hue measurement was performed in the same manner as in Test 1, the lightness ($\Delta$E) and the maximum color difference were calculated from the determined RGB values, and a graph in which the lateral axis represents the lightness ($\Delta$E) and the vertical axis represents the maximum color difference was created. FIG. 5 is a graph showing the lightness ($\Delta$E) and the maximum color difference for each number of times of filtration in the case of filtration with a commercially available filter, and FIG. 6 is a graph showing the lightness ($\Delta$E) and the maximum color difference for each number of times of filtration in the case of filtration with a deterioration inhibiting filter. The "deteriorating oil" in FIGS. 5 and 6 indicates a measurement value for rapeseed oil that has not been filtered even once.

As shown in the drawings, in both filters, the rapeseed oil has almost no color and high lightness when it is unused oil. However, as the number of times of filtration increases, the lightness decreases because the filtered rapeseed oil deteriorates and becomes brown. In addition, when the commercially available filter and the deterioration inhibiting filter are compared after filtration is performed three times, it can be seen that the rapeseed oil filtered by the deterioration inhibiting filter has a larger maximum color difference, and has inhibited deterioration.

It is difficult to visually confirm the change in hue as shown in Test 1 and Test 2, and it is difficult to determine the deterioration state of the filtration filter. However, accurate evaluation can be performed by the evaluation method according to the present invention.

Although various embodiments have been described above, it is needless to say that the present invention is not limited to these examples. It is apparent to those skilled in the art that various changes or modifications can be conceived within the scope described in the claims, and it is understood that the changes or modifications naturally fall within the technical scope of the present invention. In addition, the components described in the above embodiments may be combined in any manner without departing from the spirit of the invention.

The present application is based on a Japanese patent application (No. 2023-014037) filed on February 1, 2023, contents of which are incorporated herein by reference.

REFERENCE SIGNS LIST

1: color chart
10: base sheet
20: color sample
30: identification code
40: sample placement portion
50: rapeseed oil
51: oil tank
52: pipe
53: processing unit
54: pump

## Claims

1. A method for evaluating a filtration filter, which is a method for evaluating a filtration filter used for recycling used fats and oils, the method comprising:

    an imaging step of capturing an image of fats and oils filtered by the filtration filter or an image of the fats and oils diluted with a solvent, together with a color chart, by an imaging device;
    an image information acquisition step of acquiring image information of the imaged fats and oils or image information of the fats and oils diluted with the solvent based on image information of the imaged color chart;
    a fats and oils deterioration determination step of determining a deterioration state of the fats and oils based on the image information; and
    a filtration filter evaluation step of evaluating filtration performance and deterioration inhibiting performance of the filtration filter based on a determination result of the fats and oils deterioration determination step.

2. The method for evaluating a filtration filter according to claim 1,

    wherein the image information in the image information acquisition step is an RGB value of the fats and oils after filtration or an RGB value of the fats and oils diluted with the solvent, and
    in the fats and oils deterioration determination step, lightness ($\Delta E$) defined by the following formula (1) and a maximum color difference are calculated from the RGB value to determine a correlation between the lightness ($\Delta E$) and the maximum color difference which are defined as follows:

$$\text{Lightness } (\Delta E) = (R^2+G^2+B^2)^{0.5} \ldots (1);$$

and

    • Maximum color difference: difference between maximum value and minimum value in RGB values.

3. The method for evaluating a filtration filter according to claim 2, further comprising:

an image information correction step of correcting a white balance in the image information of the imaged fats and oils based on a white balance in the image information of the color chart, in a subsequent stage of the image information acquisition step,
wherein in the fats and oils deterioration determination step, the deterioration state of the fats and oils is determined based on the corrected image information.

4. The method for evaluating a filtration filter according to claim 3, wherein the imaging device has a white balance correction function.

5. The method for evaluating a filtration filter according to claim 4, wherein the imaging device is a digital camera or a camera-equipped mobile terminal.

6. The method for evaluating a filtration filter according to any one of claims 1 to 5, wherein the fats and oils is lubricating oil or edible oil.

FIG. 1

FIG. 2

## FIG. 3

COMMERCIALLY AVAILABLE FILTER

## FIG. 4

DETERIORATION INHIBITING FILTER

## FIG. 5

COMMERCIALLY AVAILABLE FILTER

(Graph: X-axis "LIGHTNESS" 0 to 500; Y-axis "MAXIMUM COLOR DIFFERENCE" 0 to 150. Data points labeled DETERIORATING OIL, THIRD-PASS OIL, FIRST-PASS OIL, UNUSED OIL.)

## FIG. 6

DETERIORATION INHIBITING FILTER

(Graph: X-axis "LIGHTNESS" 0 to 500; Y-axis "MAXIMUM COLOR DIFFERENCE" 0 to 150. Data points labeled DETERIORATING OIL, THIRD-PASS OIL, FIRST-PASS OIL, UNUSED OIL.)

## INTERNATIONAL SEARCH REPORT

| International application No. |
|---|
| **PCT/JP2024/002425** |

**A. CLASSIFICATION OF SUBJECT MATTER**

*G01N 21/27*(2006.01)i; *A23D 9/00*(2006.01)i; *A23D 9/02*(2006.01)i; *A47J 37/12*(2006.01)i; *B01D 24/00*(2006.01)i;
*B01D 29/00*(2006.01)i; *B01D 35/02*(2006.01)i; *F01M 11/10*(2006.01)i; *F16N 39/06*(2006.01)i; *G01J 3/46*(2006.01)i;
*G01N 33/26*(2006.01)i
FI: G01N21/27 A; G01J3/46 Z; B01D35/02 E; F16N39/06; B01D29/00 D; A47J37/12 381; F01M11/10 D; F01M11/10 Z;
G01N33/26; A23D9/02; A23D9/00 506

According to International Patent Classification (IPC) or to both national classification and IPC

**B. FIELDS SEARCHED**

Minimum documentation searched (classification system followed by classification symbols)

G01N 21/00-21/01; G01N 21/17-21/61; G01N 33/26-33/30; G01J 3/46-3/52; A23D 9/00-9/02; A47J 37/12; B01D
24/00-35/04; B01D 35/10-37/04; F01M 11/10; F16N 1/00-99/00; G08B 19/00-31/00; H04N 1/46-1/62; H04N 5/222-5/257;
H04N 7/18; H04N 23/00; H04N 23/40-23/76; H04N 23/90-23/959

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2007-301997 A (MAN ROLAND DRUCKMAS AG) 22 November 2007 (2007-11-22) paragraphs [0008], [0019], [0026]-[0027], [0036]-[0040], [0043]-[0046], [0052], [0058], fig. 1-3 | 1-6 |
| Y | WO 2022/255472 A1 (NSK LTD.) 08 December 2022 (2022-12-08) paragraphs [0014], [0084]-[0106], fig. 5-7 | 1-6 |
| A | WO 2014/204003 A1 (NABTESCO CORPORATION) 24 December 2014 (2014-12-24) entire text, fig. 1-5 | 1-6 |
| A | JP 2000-162387 A (HITACHI, LTD.) 16 June 2000 (2000-06-16) entire text, fig. 1 | 1-6 |

☑ Further documents are listed in the continuation of Box C.　　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **06 April 2024** | **16 April 2024** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**

International application No.

**PCT/JP2024/002425**

| C. DOCUMENTS CONSIDERED TO BE RELEVANT | | |
|---|---|---|
| Category* | Citation of document, with indication, where appropriate, of the relevant passages | Relevant to claim No. |
| A | JP 06-159032 A (MITSUBISHI JIDOSHA KOGYO KABUSHIKI KAISHA) 07 June 1994 (1994-06-07) entire text, fig. 1-4 | 1-6 |
| A | Microfilm of the specification and drawings annexed to the request of Japanese Utility Model Application No. 189413/1986 (Laid-open No. 093507/1988) (MEIDENSHA CORPORATION) 16 June 1988 (1988-06-16), specification, p. 9, lines 10-14, fig. 1 | 1-6 |

Form PCT/ISA/210 (second sheet) (July 2022)

## INTERNATIONAL SEARCH REPORT
### Information on patent family members

International application No.

**PCT/JP2024/002425**

| Patent document cited in search report | | | Publication date (day/month/year) | Patent family member(s) | Publication date (day/month/year) |
|---|---|---|---|---|---|
| JP | 2007-301997 | A | 22 November 2007 | EP 1854629 A1 paragraphs [0008], [0019], [0026]-[0027], [0036]-[0040], [0043]-[0046], [0052], [0058], fig. 1-3 DE 102006022204 A | |
| WO | 2022/255472 | A1 | 08 December 2022 | (Family: none) | |
| WO | 2014/204003 | A1 | 24 December 2014 | (Family: none) | |
| JP | 2000-162387 | A | 16 June 2000 | (Family: none) | |
| JP | 06-159032 | A | 07 June 1994 | (Family: none) | |
| JP | 63-093507 | U1 | 16 June 1988 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4593127 B
- JP 6006541 B

- JP 2023014037 A